# EUROPEAN PATENT APPLICATION

(11) **EP 4 818 194 A1**
(43) Date of publication of application: **07.10.2026**
(21) Application number: 24896785.3
(22) Date of filing: 02.12.2024
(51) Int. Cl.: C07K 14/00, C07K 1/04, A61K 38/16, A61P 25/00

(54) **CHEMICALLY MODIFIED POLYPEPTIDE**

(30) Priority: 01.12.2023 CN 202311639052
(71) Applicant: Suzhou Glenkol Pharma Technology Co., Ltd., Suzhou, Jiangsu, 215127 (CN); Quanzhou Haichuang Pharmaceutical Technology Co., Ltd., Quanzhou, Fujian 362221 (CN); Huiankai (Xiamen) Pharmaceutical Technology Co., Ltd., Xiamen, Fujian 361028 (CN)
(72) Inventor: CHEN, Jinhui, Suzhou, Jiangsu 215127 (CN); WU, Jie, Suzhou, Jiangsu 215127 (CN); WANG, Baoli, Suzhou, Jiangsu 215127 (CN); WANG, Hui, Suzhou, Jiangsu 215127 (CN); WANG, Xiaoting, Suzhou, Jiangsu 215127 (CN); SUN, Yanjun, Suzhou, Jiangsu 215127 (CN); ZHAO, Shu, Suzhou, Jiangsu 215127 (CN)
(74) Representative: Hautier IP - MC/EP
(86) International application number: PCT/CN2024/136127
(87) International publication number: WO 2025/113706

(57) **Abstract**

The present application provides a polypeptide compound, or a variant or a pharmaceutically acceptable salt thereof, wherein the polypeptide compound, or variant or the pharmaceutically acceptable salt thereof can be used in the preparation of a medicament for the treatment traumatic brain injury.

## Description

This application claims priority to Chinese Patent Application CN202311639052.9, filed on December 1, 2023, the disclosure of which is hereby incorporated by reference in its entirety.

### TECHNICAL FIELD

The present application belongs to the field of biomedical technology, and relates to a chemically modified polypeptide, in particular to a modified peptide useful for treating traumatic brain injury and/or inhibiting neural damage following traumatic brain injury and uses thereof.

### BACKGROUND

Traumatic brain injury (TBI) refers to an injury caused by direct or indirect external force acting on the head, and is one of the leading causes of death among children and young adults.TBI is a serious public health issue, and currently there are few measures available to reverse the initial brain injury caused by trauma. Due to the severity of the injury and limited treatment options, survivors often suffer from sequelae including motor disabilities, cognitive deficits, and epilepsy. The neurological, economic and social consequences of TBI are devastating for patients, their families and society.

According to incomplete statistics, the cumulative number of people disabled by craniocerebral injury in China is 1.5 to 2 million, with an annual increase of 80,000 to 100,000 cases. Approximately 200,000 people die from such injuries each year. Among all fatalities, about 60%-80% are caused by craniocerebral injury, and 17% result in disability due to craniocerebral injury. The cumulative number of people disabled by spinal cord injury is 500,000 to 600,000, with an annual increase of 230,000 cases, leading to an annual medical expenditure of up to 200-300 billion RMB. These statistical data demonstrate an urgent need for effective therapeutic approaches to improve functional recovery following trauma.

CN103230581A discloses the use of a short peptide sequence (KKNRNKLRRQHSY (SEQ ID NO:1)) for inhibiting neural damage following traumatic brain injury.

CN111363014A discloses a short peptide sequence (YGRKKRRQRRRKKNRNKLRRQHSY (SEQ ID NO:2)) capable of inhibiting the death of necrotic neurons following traumatic brain injury.

Despite these findings, there remains a need for short peptide sequences having one or more of the following properties: further improved polypeptide bioactivity, improved polypeptide stability, the ability to cross cell membranes, and reduced side effects.

### SUMMARY

In a first aspect, the present application provides a polypeptide compound comprising a structure represented by General Formula (I):

KX²X³RX⁵KX⁷X⁸RX¹⁰HSY (SEQ ID NO:3)-R¹ (I),

wherein X2 is selected from the group consisting of Lys, HoLys, Dap, DAB, Cit, Orn, D-Lys, D-HoLys, D-Dap, D-DAB, D-Cit, D-Orn, and Lys whose side-chain amino group is modified by Z1; X3 and X5 are each independently selected from the group consisting of Asn, Asp, D-Asn, and D-Asp; X7 is selected from the group consisting of Ile, Leu, Val, Tel, D-Ile, D-Leu, D-Val, and D-Tel; X8 is selected from the group consisting of Arg, HomoArg, D-Arg, and D-HomoArg; and X10 is selected from the group consisting of Glu, Gln, D-Glu, and D-Gln;
R¹ is selected from the group consisting of -OH, -ol, -NR²R³, and -OR⁴, wherein R² and R³ are each independently selected from the group consisting of hydrogen, C₁₋₁₆ alkyl, C₃₋₁₂ cycloalkyl, and 3- to 12-membered heterocycloalkyl, or R² and R³ together with a nitrogen atom to which R² and R³ are attached form a 3- to 12-membered heterocycloalkyl; R⁴ is selected from the group consisting of C₁₋₁₆ alkyl, C₃₋₁₂ cycloalkyl, and 3- to 12-membered heterocycloalkyl;

The alkyl, cycloalkyl, and heterocycloalkyl are each optionally substituted by one or more identical or different R⁵, where R⁵ is selected from the group consisting of halogen, nitro, cyano, hydroxy, carbonyl, carboxyl, -NRₐR_{b}, thiol, C₁₋₈ alkyl, C₁₋₈ alkoxy, halo C₁₋₈ alkyl, halo C₁₋₈ alkoxy, C₃₋₈ cycloalkyl, C₃₋₈ cycloalkyloxy, 3- to 8-membered heterocycloalkyl, -SC₁₋₃ alkyl, -SO₂C₁₋₃ alkyl, -SO₂NRₐR_{b}, -S(O)C₁₋₃ alkyl, -C(O)NRₐR_{b}, -C(O)C₁₋₃ alkyl, -C(O)OC₁₋₃ alkyl, -OC(O)C₁₋₃ alkyl, C₆₋₁₀ aryl, and 5- to 10-membered heteroaryl;
Rₐ and R_{b} are each independently hydrogen or a C₁₋₈ alkyl; or Rₐ and R_{b} together with a nitrogen atom to which Rₐ and R_{b} are attached form a 3- to 6-membered heterocycloalkyl; and
Z¹is -AEEA-COR⁶, wherein R⁶ is a C₁₋₁₅ alkyl.

In some embodiments, the polypeptide compound further includes a cell-penetrating peptide at its N-terminus or C-terminus.

In some embodiments, the cell-penetrating peptide is located at the N-terminus of the polypeptide compound, and a N-terminus of the cell-penetrating peptide is modified by R⁷. The structure of the polypeptide compound is represented by General Formula (II):

R⁷ -cell-penetrating peptide - KX²X³RX⁵KX⁷X⁸RX¹⁰HSY (SEQ ID NO:3) - R¹ (II),

wherein R⁷ is selected from the group consisting of hydrogen, C₁₋₁₆ alkyl, C₃₋₁₂ cycloalkyl, 3-to 12-membered heterocycloalkyl, R⁸CO-, and phosphate group, wherein R⁸ is selected from the group consisting of C₁₋₁₆ alkyl, C₃₋₁₂ cycloalkyl, 3- to 12-membered heterocycloalkyl, C₆₋₁₀ aryl, and 5- to 10-membered heteroaryl.

In some embodiments, the polypeptide compound includes a cell-penetrating peptide at its C-terminus, i.e., a cell-penetrating peptide is present between KX²X³RX⁵KX⁷X⁸RX¹⁰HSY(SEQ ID NO:1) and R¹.

In some embodiments, the cell-penetrating peptide of General formula (II) is selected from the group consisting of a TAT sequence, an MAP sequence, an MTS sequence, an R9 sequence, and a variant thereof.

In some embodiments, the cell-penetrating peptide of General Formula (II) is the TAT sequence or a variant thereof, wherein the TAT sequence includes at least an amino acid fragment from positions 49-57 of a transcriptional transactivator protein.

In some embodiments, the cell-penetrating peptide in the polypolypeptide compound of General Formula (II) is the TAT sequence, wherein X² is Lys; X³ and X⁵ are each Asn; X⁷ is Leu; X⁸ is Arg; and X¹⁰ is Gln. The structure of the polypeptide compound is represented by General Formula (III):

R⁷ -TAT-KKNRNKLRRQHSY(SEQ ID NO:1)-R¹ (III),

wherein TAT and KKNRNKLRRQHSY (SEQ ID NO:1) are linked via a backbone amide bond, and a nitrogen atom in the backbone amide bond is optionally C₁₋₃-alkylated, preferably methylated;

in General Formula (III), R¹ is selected from the group consisting of -OH, -ol, -NR²R³, and - OR⁴, wherein R² and R³ are each independently selected from the group consisting of hydrogen, C₁₋₁₆ alkyl, C₃₋₁₂ cycloalkyl, and 3- to 12-membered heterocycloalkyl; or R² and R³ together with a nitrogen atom to which R² and R³ are attached form a 3- to 12-membered heterocycloalkyl; and R⁴is selected from the group consisting of C₁₋₁₆ alkyl, C₃₋₁₂ cycloalkyl, and 3- to 12-membered heterocycloalkyl;
wherein the alkyl, cycloalkyl, and heterocycloalkyl are each optionally substituted by one or more identical or different R⁵;
R⁵ is selected from the group consisting of halogen, nitro, cyano, hydroxy, carbonyl, carboxyl, -NRₐR_{b}, thiol, C₁₋₈ alkyl, C₁₋₈ alkoxy, halo C₁₋₈ alkyl, halo C₁₋₈ alkoxy, C₃₋₈ cycloalkyl, C₃₋₈ cycloalkyloxy, 3- to 8-membered heterocycloalkyl, -SC₁₋₃ alkyl, -SO₂C₁₋₃ alkyl, -SO₂NRₐR_{b}, - S(O)C₁₋₃ alkyl, -C(O)NRₐR_{b}, -C(O)C₁₋₃ alkyl, -C(O)OC₁₋₃ alkyl, -OC(O)C₁₋₃ alkyl, C₆₋₁₀aryl, and 5- to 10-membered heteroaryl;
Rₐ and R_{b} are each independently hydrogen or a C₁₋₈ alkyl; or Rₐ and R_{b} together with a nitrogen atom to which Rₐ and R_{b} are attached form a 3- to 6-membered heterocycloalkyl; and when R⁷ is hydrogen, R¹ is not -OH; the TAT sequence includes at least the amino acid fragment at postitions 49-57 of the transcriptional transactivator protein;
R⁷ is selected from the group consisting of hydrogen, C₁₋₁₆ alkyl, C₃₋₁₂ cycloalkyl, 3- to 12-membered heterocycloalkyl, R⁸CO-, and phosphate group, wherein R⁸ is selected from the group consisting of C₁₋₁₆ alkyl, C₃₋₁₂ cycloalkyl, 3- to 12-membered heterocycloalkyl, C₆₋₁₀ aryl, and 5-to 10-membered heteroaryl groups.

In some embodiments, the compound of general formula (III) according to the present application may also be selected from a variant thereof or a pharmaceutically acceptable salt thereof.

In some embodiments, the present application provides a polypeptide compound, or a variant or a pharmaceutically acceptable salt thereof. The structure of the polypeptide compound is representd by General Formula (III):

R⁷ -TAT-KKNRNKLRRQHSY(SEQ ID NO:1)-R¹ (III),

wherein TAT is linked to KKNRNKLRRQHSY (SEQ ID NO:1) via a backbone amide bond, and a nitrogen atom in the backbone amide bond is optionally C₁₋₃-alkylated, preferably methylated;
R⁷ is selected from the group consisting of hydrogen, C₁₋₁₆ alkyl, C₃₋₁₂ cycloalkyl, 3- to 12-membered heterocycloalkyl, R⁸CO-, and phosphate group, wherein R⁸ is selected from the group consisting of C₁₋₁₆ alkyl, C₃₋₁₂ cycloalkyl, 3- to 12-membered heterocycloalkyl; C₆₋₁₀aryl; and 5- to 10-membered heteroaryl;
R¹is selected from the group consisting of -OH, -ol, -NR₂R₃, and -OR₄, wherein R² and R³ are each independently selected from the group consisting of hydrogen, C₁₋₁₆ alkyl, C₃₋₁₂ cycloalkyl, and 3- to 12-membered heterocycloalkyl, or R² and R³ together with a nitrogen atom to which R² and R³ are attached form a 3- to 12-membered heterocycloalkyl; and R⁴ is selected from the group consisting of C₁₋₁₆ alkyl, C₃₋₁₂ cycloalkyl, and 3- to 12-membered heterocycloalkyl;
wherein the alkyl, cycloalkyl, and heterocycloalkyl are each optionally substituted by one or more identical or different R⁵;
R⁵ is selected from the group consisting of halogen, nitro, cyano, hydroxy, carbonyl, carboxyl, -NRₐR_{b}, thiol, C₁₋₈ alkyl, C₁₋₈ alkoxy, halo C₁₋₈alkyl, halo C₁₋₈alkoxy, C₃₋₈ cycloalkyl, C₃₋₈ cycloalkyloxy, 3- to 8-membered heterocycloalkyl, -SC₁₋₃ alkyl, -SO₂C₁₋₃ alkyl, -SO₂NRₐR_{b}, - S(O)C₁₋₃ alkyl, -C(O)NRₐR_{b}, -C(O)C₁₋₃ alkyl, -C(O)OC₁₋₃alkyl, -OC(O)C₁₋₃ alkyl, C₆₋₁₀ aryl, and 5- to 10-membered heteroaryl;
Rₐ and R_{b} are each independently hydrogen or C₁₋₈ alkyl; or Rₐ and R_{b} together with a nitrogen atom to which Rₐ and R_{b} attached form a 3- to 6-membered heterocycloalkyl; and when R⁷ is hydrogen, R¹ is not -OH; The TAT sequence includes at least the amino acid fragment from positions 49 to 57 of the transcriptional transactivator protein.

In some embodiments, the TAT sequence is selected from the group consisting of the short peptides YGRKKRRQRRR (SEQ ID NO:4), GRKKRRQRRR (SEQ ID NO:33), YGRKKRRQRRRPPQ (SEQ ID NO:34), and GRKKRRQRRRQ (SEQ ID NO:35).

In some embodiments, the variant of TAT sequence includes 1, 2, or 3 amino acid substitutions, additions, or deletions relative to the TAT sequence.

In some specific embodiments, the variant of the TAT sequence includes a sequence having at least 80%, at least 85%, at least 90%, at least 95%, at least 99%, or 100% identity to YGRKKRRQRRR (SEQ ID NO:4).

In some embodiments, the variant of the TAT sequence is an amino acid sequences formed by replacing at least one Gln in the TAT sequence by Glu, and/or at least one L-Arg in the TAT sequence by D-Arg, and/or at least one L-Lys in the TAT sequence by D-Lys.

In some embodiments, R¹ is selected from the group consisting of -OH and -NR²R³, wherein R² and R³ are each independently selected from the group consisting of hydrogen and C₁₋₃ alkyl.

In some embodiments, R¹is selected from the group consisting of -OH and -NH₂.

In some embodiments, R¹ is -OH.

In some embodiments, R¹ is -NH₂.

In some embodiments, R⁷ is selected from the group consisting of hydrogen, methyl, acetyl, and phosphate group.

In some embodiments, R⁷ in the polypeptide compound is selected from the group consisting of hydrogen, C₁₋₃ alkyl, and acetyl.

In some embodiments, R⁷ is hydrogen.

In some embodiments, R⁷ is methyl.

In some embodiments, R⁷ is phosphate group.

In some embodiments, the polypeptide compound is selected from the polypeptide compounds shown in SEQ ID NO:5 to SEQ ID NO:29:
Me-YGRKKRRQRRRKKNRNKLRRQHSY (SEQ ID NO:5),
pYGRKKRRQRRRKKNRNKLRRQHSY (SEQ ID NO:6),
Ac-YGRKKRRQRRRKKNRNKLRRQHSY (SEQ ID NO:7),
C₁₅H₃₁CO-YGRKKRRQRRRKKNRNKLRRQHSY (SEQ ID NO:8),
YGRKKRRQRRRKKNRNKLRRQHSY-NH₂ (SEQ ID NO:9),
Ac-YGRKKRRQRRRKKNRNKLRRQHSY-NH₂ (SEQ ID NO:10),
pYGRKKRRQRRRKKNRNKLRRQHSY-NH_{2 (}SEQ ID NO:11),
Me-YGRKKRRQRRRKKNRNKLRRQHSY-NH₂ (SEQ ID NO:12),
C₁₅H₃₁CO-YGRKKRRQRRRKKNRNKLRRQHSY-NH₂(SEQ ID NO:13),
YGRKKRRQRRRKKNRNKIRRQHSY (SEQ ID NO:14),
YGRKKRRQRRRKKNRNKIRRQHSY-NH₂ (SEQ ID NO:15),
YGRKKRRQRRRKKDRDKLRRQHSY (SEQ ID NO:16),
YGRKKRRQRRR-K(N-AEEA-Palmitic acid)-KNRNKLRRQHSY (SEQ ID NO:17),
YGRKKRRQRRRKKNRNKLRREHSY (SEQ ID NO:18),
YGRKKRRQRRRKKNRDKLRREHSY (SEQ ID NO:19),
YGRKKRRERRRKKDRNKLRRQHSY (SEQ ID NO:20),
Ac-YGRKKRRERRRKKDRDKLRREHSY-NH₂ (SEQ ID NO:21),
Me-YGRKKRRERRRKKDRDKLRREHSY-NH₂ (SEQ ID NO:22),
Ac-YGRKKRRERRRKKDRDK(D-L)RREHSY-NH₂(SEQ ID NO:23),
Ac-YGRKKRRERRRKKDRDKLRRQHSY-NH₂ (SEQ ID NO:24),
Me-YGRKKRRERRRKKDRDKLRRQHSY-NH₂ (SEQ ID NO:25),
Ac-YGRKKRRERRRKKDRDKVRREHSY-NH₂ (SEQ ID NO:26),
Me-YAibRKKRRERRRKKDRDKTleRREHSY-NH₂ (SEQ ID NO:27),
Ac-YGRKKRRERRRKKDRDKTleRREHSY-NH₂ (SEQ ID NO:28) and
Ac-YGRK(D-K)(D-R)RER(D-R)RKKDRDK(D-L)(D-R)REHSY-NH₂ (SEQ ID NO:29).

In a second aspect, the present application provides an isolated polynucleotide encoding the polypeptide compound according to the present application.

In a third aspect, the present application provides a plasmid including the polynucleotide described above.

In a fourth aspect, the present application provides an expression vector including the polynucleotide described above.

In a fifth aspect, the present application provides a host cell including the aforementioned expression vector or plasmid, or having integrated into its genome an exogenous isolated polynucleotide as described above.

In a sixth aspect, the present application provides a pharmaceutical composition, including the aforementioned polypeptide compound, or a variant or a pharmaceutically acceptable salt thereof, and a pharmaceutically acceptable excipient.

In a seventh aspect, the present application provides the use of the aforementioned polypeptide compound, or a variant or a pharmaceutically acceptable salt thereof, or the aforementioned pharmaceutical composition in the manufacture of a medicament. In some embodiments, the medicament is for the treatment and/or prevention of traumatic brain injury and/or for inhibiting neural damage following traumatic brain injury.

In an eighth aspect, the present application provides a method for treating and/or preventing traumatic brain injury and/or inhibiting neural damage following traumatic brain injury, including administering the aforementioned polypeptide compounds, or a variant or a pharmaceutically acceptable salt thereof, or the pharmaceutical composition described in the sixth aspect to a subject.

In a ninth aspect, the present application provides the aforementioned pharmaceutical composition for use in treating and/or preventing traumatic brain injury and/or in inhibiting neural damage following traumatic brain injury.

Without being bound by theory, the inventors of the present application have discovered that certain specific modifications in the polypeptide compounds provided herein can significantly improve the stability of the modified polypeptide, and/or reduce glutamate-induced excitatory death of nerve cells and enhance neural cell viability, thereby significantly improving the efficacy of the polypeptides according to the present application in treating traumatic brain injury and inhibiting nerve damage following traumatic brain injury.

### DETAILED DESCRIPTION

The technical solutions according to the present application are further illustrated below through specific examples. Those skilled in the art should understand that the examples are provided merely for facilitating understanding the present application and should not be construed as limitations to the present application.

For a clearer understanding of the technical content of the present application, the terms used herein are further defined below.

"Variant" refers to an amino acid sequence in which one or more amino acids have been altered. A variant may have "conservative" changes, wherein the substituted amino acid has similar structural or chemical properties, such as replacement of leucine (L) with isoleucine (I), replacement of aspartamide (N) with aspartic acid (D), or replacement of glutamine (Q) with glutamic acid (E). A variant may have "non-conservative" changes, such as replacement of leucine (L) with valine (V), replacement of glycine (G) with tryptophan (W), or replacement of a natural amino acid with a non-natural amino acid. Optionally, the variant may have replacement of leucine (L) with L-terc-leucine (Tle), or replacement of glycine (G) with 2-aminoisobutyric acid (Aib). Similar minor alterations may also include amino acid deletions and/or insertions.

"Y-ol" refers to tyrosinol.

Amino acids are linked together by amide bond to form covalent peptide bond linking a backbone carboxylic acid group of one amino acid with a backbone amino group of another amino acid, thereby forming a peptide bond (-C(=O)-NH-) or backbone amide bond.

"Alkyl" refers to a fully saturated aliphatic hydrocarbon group. C₁₋₁₆ alkyl refers to a straight-chain or branched alkyl group having 1 to 16 carbon atoms in the chain.C₁₋₆ alkyl refers to a straight-chain or branched alkyl group having 1 to 6 carbon atoms in the chain. Examples of alkyl groups include, but are not limited to, methyl, ethyl, n-propyl, isopropyl, butyl, isobutyl, sec-butyl, tert-butyl, pentyl, isopentyl, tert-pentyl, hexyl, and isohexyl.

"Cycloalkyl" refers to saturated or partially saturated monocyclic, fused, bridged, or spirocyclic ring in which each carbocyclic ring contains at least 3 ring atoms. C₃₋₁₈ cycloalkyl means that each carbocyclic ring has 3 to 18 ring atoms. C₃₋₆ cycloalkyl means that each carbocyclic ring has 3 to 6 ring atoms. Exemplary instances of cycloalkyl include, but are not limited to, the following moieties:

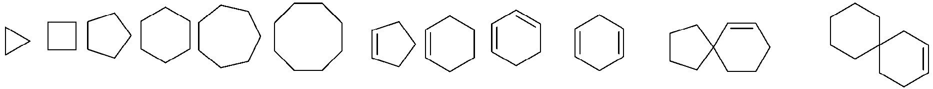

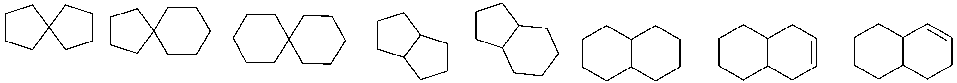

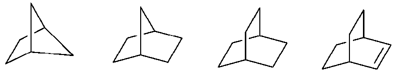

"Heterocycloalkyl" refers to a saturated or partially saturated monocyclic, fused polycyclic, bridged, or spirocyclic structure having at least one heteroatom selected from nitrogen, oxygen, and sulfur in the ring structure. Exemplary instances of heterocyclic include, but are not limited to, the following moieties:

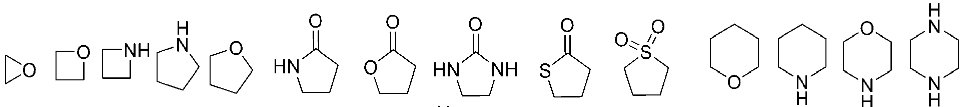

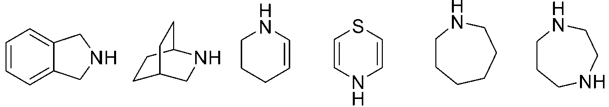

"Aryl" refers to an all-carbon monocyclic or fused polycyclic (i.e., rings sharing adjacent carbon atom pairs) group with a conjugated π-electron system. "C₆₋₁₀ aryl" refers to a monocyclic or bicyclic aryl group having 6 to 10 carbon atoms. Exemplary instances of aryl include, but are not limited to, the following moieties: phenyl, naphthyl, and the like.

"Heteroaryl" refers to a monocyclic, bicyclic, or polycyclic 4n+2 aromatic ring system (e.g., having 6 or 10 π electrons shared in a cyclic arrangement) containing ring carbon atoms and ring heteroatoms, wherein each heteroatom is independently selected from the group consisting of nitrogen, oxygen, and sulfur. Exemplary instances of heteroaryl include, but are not limited to, the following moieties: thienyl, Thienyl, furanyl, thiazolyl, isothiazolyl, imidazolyl, pyrrolyl, pyrazolyl, pyridinyl, pyridazinyl, pyrimidinyl, pyrazinyl, and the like.

"Halogen" refers to fluorine, chlorine, bromine, or iodine.

"Halo" means that one or more (e.g., 1, 2, 3, 4, or 5) hydrogen atoms in a group are replaced by halogen atoms.

The correspondence between amino acid abbreviations and their full names is shown in Table 1.

**Table 1: Amino Acid Abbreviations and Corresponding Full Names**

| Abbreviation | Full Name |
|---|---|
| Ala or A | Alanine |
| Arg or R | Arginine |
| Asn or N | Asparagine |
| Asp or D | Aspartic acid |
| Cys or C | Cysteine |
| Glu or E | Glutamic acid |
| Gln or Q | Glutamine |
| Gly or G | Glycine |
| His or H | Histidine |
| Ile or I | Isoleucine |
| Leu or L | Leucine |
| Lys or K | Lysine |
| Met or M | Methionine |
| Phe or F | Phenylalanine |
| Pro or P | Proline |
| Ser or S | Serine |
| Thr or T | Threonine |
| Trp or W | Tryptophan |
| Tyr or Y | Tyrosine |
| Val or V | Valine |

HoLys represents hydroxylysine, whose full name is 5-hydroxylysine.

Dap represents 2,6-diaminoheptanedioic acid.

DAB represents 2,4-diaminobutyric acid.

Cit represents citrulline.

Orn represents ornithine.

HomoArg represents homoarginine.

The amino acid residues in the peptide sequences according to the present application are represented by amino acid abbreviations.

As used herein, "Ac" and "NH₂" refer to the acetyl and amide-terminus, respectively.

Unless otherwise indicated, the amino acids described in the present application are "L-amino acids," represented by a single uppercase letter or a three-letter abbreviations of the amino acid. "D-amino acids" in the present application are represented by a single lowercase letter of the amino acid abbreviations, by a three-letter amino acid abbreviation prefixed with the letter "D", or by an uppercase single-letter amino acid abbreviation prefixed with the letter "D". For example, "L-leucine" may be represented by "Leu" or "L," while "D-leucine" may be represented by "D-Leu," represented by "l," or by "D-L."

Aib represents 2-aminoisobutyric acid.

Tle represents L-terc-leucine.

In one embodiment, the amino acid fragment sequence from positions 49 to 57 of the transcriptional transactivator protein is RKKRRQRRR (SEQ ID NO: 36).

As used herein, "treatment", "treating" or "treat" refers to the administration of an effective amount of a polypeptide compound according to the present application to an individual to alleviate symptoms or complications, delay a disease, impede the progression of a condition, mitigate or relieve symptoms and complications, and/or cure or eliminate a disease or condition, as well as to prevent a condition, wherein prevention should be understood as the administration of the polypeptide compound according to the present application to an individual for the purpose of combating a disease or condition to prevent the onset of symptoms or complications. In one embodiment, "treatment", "treating" or "treat" refers to the administration of an effective amount of the polypeptide compound according to the present application for the purpose of alleviating, ameliorating, impeding, or eradicating (curing) symptoms or a condition.

As used herein, "effective amount" or "therapeutically effective amount" refers to a sufficient amount of a drug or agent that is sufficient to achieve the desired therapeutic effect.

The peptides according to the present application:
The peptides according to the present application may further include, at their N-terminus or C-terminus, a cell-penetrating peptide capable of guiding the peptide according to the present application across the blood-brain barrier and neural cell membranes. Suitable cell-penetrating peptides known in the art can be used in the present application, provided that they are capable of guiding the peptide according to the present application across the blood-brain barrier and neural cell membranes. Exemplary cell-penetrating peptides may be selected from TAT sequence (selected from "YGRKKRRQRRR (SEQ ID NO:4)", "GRKKRRQRRR (SEQ ID NO:33)", "YGRKKRRQRRRPPQ (SEQ ID NO: 34)", or "GRKKRRQRRRQ (SEQ ID NO: 35)"), model amphipathic peptide (MAP) sequences ("KLALKLALKALKAALKLA (SEQ ID NO: 30)"), membrane translocating peptide (MTS) sequence ("AAVALLPAVLLALLAP (SEQ ID NO:31)") or R9 ("RRRRRRRRR (SEQ ID NO:32)") sequence. In a particularly preferred embodiment, the cell-penetrating peptide according to the present application is the TAT sequence. Optionally, the peptide according to the present application may be chemically modified to facilitate its passage across the BBB and neural cell membranes, including but not limited to amidation, acetylation, phosphorylation, side-chain modification, palmitoylation, variants, and the like.

"Pharmaceutically acceptable salt" refers to a salt obtained by reacting a compound with a pharmaceutically acceptable (relatively non-toxic, safe, and suitable administration to a subject) acid or base. When the compound contains a relatively acidic functional group, a base addition salt may be obtained by contacting the free form of the compound with an adequate amount of a pharmaceutically acceptable base in a suitable inert solvent. Pharmaceutically acceptable base addition salts include, but are not limited to, sodium, potassium, calcium, aluminum, magnesium, bismuth, and ammonium salts. When a compound contains a relatively basic functional group, a acid addition salt can be obtained by contacting the free form of the compound with an adequate amount of a pharmaceutically acceptable acid in a suitable inert solvent. Pharmaceutically acceptable acid addition salts include, but are not limited to, hydrochloride, sulfate, and mesylate salts.

"Pharmaceutical composition" refers to a mixture of one or more of the compounds disclosed herein or a pharmaceutically acceptable salts thereof, with other chemical components, such as pharmaceutically acceptable carriers and excipients. The purpose of a pharmaceutical composition is to facilitate the administration of the compound to a biological entity.

Non-limiting examples of "excipients" include binders, disintegrants, lubricants, glidants, stabilizers, fillers, and diluents.

EC₅₀ is the half-maximal effective concentration, meaning the drug concentration required to produce 50% of the maximum effect.

mmol represents millimole.

eq represents equivalent, referring to the molar ratio of two reagents or materials.

DIC represents N,N'-diisopropylcarbodiimide.

TBTU represents O-(Benzotriazol-1-yl)-N,N,N',N'-tetramethyluronium tetrafluoroborate.

DIPEA represents N,N-diisopropylethylamine.

Fmoc group represents fluorenylmethyloxycarbonyl.

2-Cl-CTC-Resin: chloro (o-chlorophenyl) diphenylmethane.

Fmoc-Tyr(tBu)-OH: N- fluorenylmethyloxycarbonyl -O-tert-butyl-L-tyrosine.

Fmoc-Ser(tBu)-OH: N- fluorenylmethyloxycarbonyl -O-tert-butyl-L-serine.

Fmoc-His(Trt)-OH: N- fluorenylmethyloxycarbonyl -N'-trityl-L-histidine.

Fmoc-Gln(Trt)-OH: N- fluorenylmethyloxycarbonyl -N'- trityl -L-glutamine.

Fmoc-Leu-OH: N- fluorenylmethyloxycarbonyl -L-leucine.

Fmoc-Lys(Boc)-OH: N- fluorenylmethyloxycarbonyl-N-tert-butoxycarbonyl-L-lysine.

Fmoc-Asn(Trt)-OH: fluorenylmethyloxycarbonyl-N-trityl-L-aspartamide.

Fmoc-Arg(Pbf)-OH: N-fluorenylmethoxycarbonyl-2,2,4,6,7-pentamethyldihydrobenzofuran- 5-sulfonyl-L-arginine.

Fmoc-Gln(Trt)-OH: N-fluorenylmethoxycarbonyl-N'-trityl-L-glutamine.

Fmoc-Gly-OH: N-fluorenylmethoxycarbonyl-glycine.

Fmoc-N-Me-Tyr(tBu)-OH: O-tert-butyl-N-[(9H-fluoren-9-ylmethoxy)carbonyl]-N-methyl-L-tyrosine.

Fmoc-Tyr(H₂PO₃)-OH: N-fluorenylmethoxycarbonyl-O-phospho-L-tyrosine.

TFA represents trifluoroacetic acid.

MPA represents 2-mercaptopropionic acid.

TIS represents triisopropylsilane.

HOBt represents 1-hydroxybenzotriazole.

MK-801: Dizocilpine maleate (maleate), an effective, selective, non-competitive NMDA receptor antagonist.

### Material Sources:

All amino acid materials were purchased from GL Biochem (Shanghai) Ltd.; the coupling agents DIC, HOBT, and TBTU were purchased from Suzhou Highfine Biotech Co., Ltd.

DMEM/F12 medium was purchased from Biosharp.

Fetal bovine serum was purchased from Suzhou ExCell Bio Co., Ltd.

Penicillin/streptomycin mixed solution was purchased from Hyclone.

Glutamic acid was purchased from Sigma.

Propidium iodide/Hoechst staining solution was purchased from Sigma.

4% paraformaldehyde was purchased from Macklin.

Phosphate-buffered saline was purchased from Biosharp.

The correspondence between example numbers and amino acid sequence numbers is shown in Table 2:

**Table 2: Correspondence Table of Example Numbers and Amino Acid Sequence Numbers**

| Example | Structure (Sequence No.) |
|---|---|
| Example 1 | YGRKKRRQRRRKKNRNKLRRQHSY (SEQ ID NO:2) |
| Example 2 | Me-YGRKKRRQRRRKKNRNKLRRQHSY (SEQ ID NO:5) |
| Example 3 | pYGRKKRRQRRRKKNRNKLRRQHSY (SEQ ID NO:6) |
| Example 4 | Ac-YGRKKRRQRRRKKNRNKLRRQHSY (SEQ ID NO:7) |
| Example 5 | C₁₅H₃₁CO-YGRKKRRQRRRKKNRNKLRRQHSY (SEQ ID NO:8) |
| Example 6 | YGRKKRRQRRRKKNRNKLRRQHSY-NH₂ (SEQ ID NO:9) |
| Example 7 | Ac-YGRKKRRQRRRKKNRNKLRRQHSY-NH₂ (SEQ ID NO:10) |
| Example 8 | pYGRKKRRQRRRKKNRNKLRRQHSY-NH₂ (SEQ ID NO:11) |
| Example 9 | Me-YGRKKRRQRRRKKNRNKLRRQHSY-NH₂ (SEQ ID NO:12) |
| Example 10 | C₁₅H₃₁CO-YGRKKRRQRRRKKNRNKLRRQHSY-NH₂ (SEQ ID NO:13) |
| Example 11 | YGRKKRRQRRRKKNRNKIRRQHSY (SEQ ID NO:14) |
| Example 12 | YGRKKRRQRRRKKNRNKIRRQHSY-NH₂ (SEQ ID NO:15) |
| Example 13 | YGRKKRRQRRRKKDRDKLRRQHSY (SEQ ID NO:16) |
| Example 14 | YGRKKRRQRRRK(N-AEEA-Palmitic acid)KNRNKLRRQHSY (SEQ ID NO:17) |
| Example 15 | YGRKKRRQRRRKKNRNKLRREHSY (SEQ ID NO:18) |
| Example 16 | YGRKKRRQRRRKKNRDKLRREHSY (SEQ ID NO:19) |
| Example 17 | YGRKKRRERRRKKDRNKLRRQHSY (SEQ ID NO:20) |
| Example 18 | Ac-YGRKKRRERRRKKDRDKLRREHSY-NH₂ (SEQ ID NO:21) |
| Example 19 | Me-YGRKKRRERRRKKDRDKLRREHSY-NH₂ (SEQ ID NO:22) |
| Example 20 | Ac-YGRKKRRERRRKKDRDK(D-L)RREHSY-NH₂ (SEQ ID NO:23) |
| Example 21 | Ac-YGRKKRRERRRKKDRDKLRRQHSY-NH₂ (SEQ ID NO:24) |
| Example 22 | Me-YGRKKRRERRRKKDRDKLRRQHSY-NH₂ (SEQ ID NO:25) |
| Example 23 | Ac-YGRKKRRERRRKKDRDKVRREHSY-NH₂ (SEQ ID NO:26) |
| Example 24 | Me-YAibRKKRRERRRKKDRDKTleRREHSY-NH₂ (SEQ ID NO:27) |
| Example 25 | Ac-YGRKKRRERRRKKDRDKTleRREHSY-NH₂ (SEQ ID NO:28) |
| Example 26 | Ac-YGRK(D-K)(D-R)RER(D-R)RKKDRDK(D-L)(D-R)REHSY-NH₂ (SEQ ID NO:29) |

### Preparation Examples:

### Example 1: Preparation of YGRKKRRQRRRKKNRNKLRRQHSY (SEQ ID NO: 2) polypeptide

YGRKKRRQRRRKKNRNKLRRQHSY (SEQ ID NO: 2) was prepared according to the method disclosed in CN111363014A.

### Example 2: Synthesis of Me-YGRKKRRQRRRKKNRNKLRRQHSY (SEQ ID NO: 5)

Taking a synthesis scale of 1 mmol as an example.

### 2.1 Synthesis of Fmoc-Tyr(tBu)-CTC-Resin

3.0 g of 2-Cl-CTC-Resin with a substitution degree of 1.08 mmol/g was weighed and added to a peptide reaction column, washed once with 10 mL of *N,N*-dimethylformamide, swelled with 20 mL of dichloromethane for 20 minutes, and the solution was removed by suction.

1.4 eq of Fmoc-Tyr(tBu)-OH and 4.2 eq of *N,N*-diisopropylethylamine were weighed and added to the peptide reaction column, followed by 20 mL of *N,N*-dimethylformamide. The reaction was carried out at a constant temperature of 30°C for three hours. The solution was removed by suction, and the resin was washed three times with *N,N*-dimethylformamide, then capped twice with 20 mL of a capping solution consisting of dichloromethane/*methanol*/*N,N-*diisopropylethylamine in a ratio of 17:2:1, for 20 minutes each time. After completion, the resin was washed four times with *N,N*-dimethylformamide, shrunk three times with methanol, and dried under vacuum.

1 mmol obtained resin was weighed and swelled in *N,N*-dimethylformamide for 30 minutes. The protecting group was removed twice with a 20% piperidine/*N,N*-dimethylformamide solution (20 mL each time), for 5 and 10 minutes, respectively. After completion, the resin was washed six times with 20 mL of *N,N*-dimethylformamide each time. The peptide reaction column was maintained at a constant temperature of 30°C during the reaction and washing processes. A small amount of resin was taken for the ninhydrin test. The resin turned blue-black.

### 2.2 Coupling of Fmoc-Ser(tBu)-OH

Fmoc-Ser(tBu)-OH (4 eq), TBTU (4 eq), and *N,N*-diisopropylethylamine (6 eq) were weighed and added to the peptide reaction column, followed by 20 mL of *N,N*-dimethylformamide. The reaction was carried out at a constant temperature of 30°C for two hours. The solution was removed by suction, and the resin was washed three times with 20 mL of *N,N*-dimethylformamide each time. A small amount of the resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with 20 mL of *N,N-*dimethylformamide each time. A small amount of resin was taken for ninhydrin test. The resin turned blue-black, indicating complete deprotection.

### 2.3 Coupling of Fmoc-His(Trt)-OH

Fmoc-His(Trt)-OH (4 eq), HOBt (4 eq), and DIC (6 eq) were weighed and added to a peptide reaction column, followed by 20 mL of *N,*N-dimethylformamide. The reaction was allowed to proceed for two hours at a constant temperature of 30°C. The solution was removed by suction and the resin was washed three times with *N,N*-dimethylformamide (20 mL each time). A small amount of the resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.4 Coupling of Fmoc-Gln(Trt)-OH

Fmoc-Gln(Trt)-OH (4 eq), HOBt (4 eq), and DIC (6 eq) were weighed and added to a peptide reaction column, followed by 20 mL of *N,N*-dimethylformamide. Teaction was allowed to proceed for 2 hours at a constant temperature of 30°C. The solution was removed by suction and the resin was washed three times with *N,N*-dimethylformamide (20 mL each time). A small amount of resin was taken for ninhydrin test. the resin was transparent and colorless indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.5 Coupling of Fmoc-Arg(Pbf)-OH

Fmoc-Arg(Pbf)-OH (4 eq), HOBt (4 eq), and DIC (6 eq) were weighted and added to a peptide reaction column, followed by 20 mL of *N,*N-dimethylformamide. The reaction was allowed to proceed for two hours at a constant temperature of 30°C. The solution was removed by suction and the resin was washed three times with *N,N*-dimethylformamide (20 mL each time). A small amount of resin was taken for ninhydrin test. The resin is transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.6 Coupling of Fmoc-Arg(Pbf)-OH

Fmoc-Arg(Pbf)-OH (4 eq), HOBt (4 eq), and DIC (6 eq) were weighed and added to a peptide reaction column, followed by 20 mL of *N,N*-dimethylformamide. The reaction was allowed to perceed for 2 hours at a constant temperature of 30°C. The solution was removed by suction and the resin was washed three times with *N,*N-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the reain was washed six times with *N,*N-dimethylformamide (20 mL each time). A small amount of resin was taken for ninhydrin test. The resin turned blue-black, indicating complete deprotection.

### 2.7 Coupling of Fmoc-Leu-OH

Fmoc-Leu-OH (4 eq), HOBt (4 eq), and DIC (6 eq) were weighed and added to a peptide reaction column, followed by 20 mL of *N,N*-dimethylformamide. The reaction was allowed to proceed for two hours at a constant temperature of 30°C. The solution was removed by suction and the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the reasin was washed six times with *N,N*-dimethylformamide (20 mL each time). A small amount of resin was taken for ninhydrin test. The resin was blue-black, indicating complete deprotection.

### 2.8 Coupling of Fmoc-Lys(Boc)-OH

Fmoc-Lys(Boc)-OH (4 eq), HOBt (4 eq), and DIC (6 eq) were weighed and added to the peptide reaction column, followed by 20 mL of *N,N*-dimethylformamide. The reaction was allowed to proceed for 2 hours at a constant temperature of 30°C. The solution was removed by suction and the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.9 Coupling of Fmoc-Asn(Trt)-OH

Fmoc-Asn(Trt)-OH (4 eq), HOBt (4 eq), and DIC (6 eq) were weighed and added to a peptide reaction column, followed by 20 mL of *N,N*-dimethylformamide. The reaction was allowed to proceed for two hours at a constant temperature of 30°C. The solution was removed by suction and the resin was washed three times with *N,N*-dimethylformamide (20 mL each time). A small amount of resin was taken for ninhydrin test. The resin was transparent and colorless, indicating complete reaction.

The peptide reaction columnwas maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.10 Coupling of Fmoc-Arg(Pbf)-OH

Fmoc-Arg(Pbf)-OH (4 eq), HOBt (4 eq), and DIC (6 eq) were weighed and added to a peptide reaction column, followd by 20 mL of *N,N*-dimethylformamide. The reaction was allowed to proceed for 2 hours at a constant temperature of 30°C. The solution was removed by suction and the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution ( 20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.11 Coupling of Fmoc-Asn(Trt)-OH

Fmoc-Asn(Trt)-OH (4 eq), HOBt (4 eq), and DIC (6 eq) were weighed and added to a peptide reaction column, followed by 20 mL of *N,N*-dimethylformamide. The reaction was allowed to proceed for two hours at a constant temperature of 30°C. The solution was removed by suction and the resin was washed three times with *N,*N-dimethylformamide (20 mL each time). A small amount of resin was taken for ninhydrin test. The resin is transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.12 Coupling of Fmoc-Lys(Boc)-OH

Fmoc-Lys(Boc)-OH (4 eq), HOBt (4 eq), and DIC (6 eq) were weighed and added to a peptide reaction column, followed by 20 mL of *N,N*-dimethylformamide. The reaction was allowed to proceed for two hours at a constant temperature of 30°C. The solution was removed by suction and the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.13 Coupling of Fmoc-Lys(Boc)-OH

Fmoc-Lys(Boc)-OH (4 eq), HOBt (4 eq), and DIC (6 eq) were weighed and added to a peptide reaction column, followed by 20 mL of *N,N*-dimethylformamide. The reaction was allowed to proceed for two hours at a constant temperature of 30°C. The solution was removed by suction and the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.14 Coupling of Fmoc-Arg(Pbf)-OH

Fmoc-Arg(Pbf)-OH (4 eq), HOBt (4 eq), and DIC (6 eq) were weighed and added to a peptide reaction column, followed by 20 mL of *N,N*-dimethylformamide. The reaction was allowed to proceed for 2 hours at a constant temperature of 30°C. The solution was removed by suction and then the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.15 Coupling of Fmoc-Arg(Pbf)-OH

Fmoc-Arg(Pbf)-OH (4 eq), HOBt (4 eq), and DIC (6 eq) were weighed and added to a peptide reaction column, followed by 20 mL of *N,N*-dimethylformamide. The reaction was allowed to proceed for two hours at a constant temperature of 30°C. The solution was removed by suction and then the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution ( 20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 16 Coupling of Fmoc-Arg(Pbf)-OH

Fmoc-Arg(Pbf)-OH (4 eq), HOBt (4 eq), and DIC (6 eq) were weighed and added to a peptide reaction column followed by 20 mL of *N,N*-dimethylformamide. The reaction was allowed to proceed for 2 hours at a constant temperature of 30°C. The solution was removed by suction and then the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% *piperidine*/*N,N*-dimethylformamide solution (20 mL each time), for 5 and 10 minutes, respectively. After completion, the resin was washed six times with *N,N*-dimethylformamide (20 mL each time). A small amount of resin for ninhydrin test The resin turned blue-black, indicating complete deprotection.

### 2.17 Coupling of Fmoc-Gln(Trt)-OH

Fmoc-Gln(Trt)-OH (4 eq), HOBt (4 eq), and DIC (6 eq) were weighed and added to a peptide reaction column, followed by 20 mL of *N,N*-dimethylformamide. The reaction was allowed to proceed for two hours at a constant temperature of 30°C. The solution was removed by suction and then the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.18 Coupling of Fmoc-Arg(Pbf)-OH

Fmoc-Arg(Pbf)-OH (4 eq), HOBt (4 eq), and DIC (6 eq) were weighed and added to the peptide reaction column, followed by 20 mL of *N,N*-dimethylformamide. The reaction was allowed to proceed for two hours at a constant temperature of 30°C. The solution was removed by suction and then the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.19 Coupling of Fmoc-Arg(Pbf)-OH

Fmoc-Arg(Pbf)-OH (4 eq), HOBt (4 eq), and DIC (6 eq) were weighed and added to a peptide reaction column, followed by 20 mL of *N,N*-dimethylformamide. The reaction was allowed to proceed for two hours at a constant temperature of 30°C. The solution was removed by suction and then the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.20 Coupling of Fmoc-Lys(Boc)-OH

Fmoc-Lys(Boc)-OH (4 eq), HOBt (4 eq), and DIC (6 eq) were weighed and added to a peptide reaction column, followed by 20 mL of *N,N*-dimethylformamide. The reaction was allowed to proceed for 2 hours at a constant temperature of 30°C. The solution was removed by suction and then the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.21 Coupling of Fmoc-Lys(Boc)-OH

Fmoc-Lys(Boc)-OH (4 eq), HOBt (4 eq), and DIC (6 eq) were weighed and added to a peptide reaction column, followed by 20 mL of *N,N*-dimethylformamide. The reaction was allowed to proceed for two hours at a constant temperature of 30°C. The solution was removed by suction and then the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.22 Coupling of Fmoc-Arg(Pbf)-OH

Fmoc-Arg(Pbf)-OH (4 eq), HOBt (4 eq), and DIC (6 eq) were weighed and added to a peptide reaction column, followed by 20 mL of *N,N*-dimethylformamide. The reaction was allowed to proceed for 2 hours at a constant temperature of 30°C. The solution was removed by suction and then the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection.

### 2.23 Coupling of Fmoc-Gly-OH

Fmoc-Gly-OH (4 eq), HOBt (4 eq), and DIC (6 eq) were weighed and added to a peptide reaction column, followed by 20 mL of *N,N*-dimethylformamide. The reaction was allowed to proceed for two hours at a constant temperature of 30°C. The solution was removed by suction and then the resin was washed three times with *N,N*-dimethylformamide (20 mL each time). A small amount of resin was taken for ninhydrin test. The resin is clear and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide, 20 mL each time. A small amount of resin was taken for ninhydrin test. The resin turned blue-black, indicating complete deprotection.

### 2.24 Coupling of Fmoc-N-Me-Tyr(tBu)-OH

Fmoc-N-Me-Tyr(tBu)-OH (4 eq), HOBt (4 eq), and DIC (6 eq) were weighed and added to a peptide reaction column, followed by 20 mL of *N,N*-dimethylformamide. The reaction was allowed to proceed for two hours at a constant temperature of 30°C. The solution was removed by suction and then the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection. The resin was shrunk with methanol three times (20 mL, 3 minutes each time) and dried under vacuum to obtain the peptide resin.

### 2.25 Cleavage of the Peptide Resin

An ice-cold cleavage cocktail (TFA/MPA/TIS/H₂O = 90:5:2.5:2.5), in a volume equivalent to 10 times the weight of the peptide resin, was added to the flask containing the peptide resin. The reaction was carried out at a constant temperature of 25 °C for 2 hours. After filtration, the filtrate was added to ice-cold methyl tert-butyl ether with a volume of 10 equivalents relative to the cleavage cocktail, and precipitated for 10 minutes, followed by centrifugation. The precipitate was washed repeatedly with room-temperature methyl tert-butyl ether four times and dried under vacuum (< 50 °C) for 3 hours to obtain the crude peptide.

### 2.26 Purification

Mobile phase: water containing 0.1% trifluoroacetic acid/acetonitrile containing 0.1% trifluoroacetic acid; preparative column: ODS-PK C18, 30 mm × 250 mm × 8 mm; flow rate: 25 mL/min; column temperature: 25 °C; wavelength: 220 nm.

Gradient: water/acetonitrile: 5:95 to 95:5, 40 min.

The collected sample was concentrated under reduced pressure and lyophilized to obtain the pure peptide. ESI-MS m/z [M+1]⁺: 3282.94.

### Example 3: Synthesis of pYGRKKRRQRRRKKNRNKLRRQHSY (SEQ ID NO: 6)

The preparation was performed according to the procedure of Example 2, except that Fmoc-N-Me-Tyr(tBu)-OH in Step 2.24 was replaced with Fmoc-Tyr(H₂PO₃Bzl)-OH.

### 3.1 Purification

Mobile phase: water containing 0.1% trifluoroacetic acid/acetonitrile containing 0.1% trifluoroacetic acid; preparative column: ODS-PK C18, 30 mm × 250 mm × 8 mm; flow rate: 25 mL/min; column temperature: 25°C; wavelength: 220 nm.

Gradient: water/acetonitrile: 5:95 to 95:5, 40 min.

The collected sample was concentrated under reduced pressure and lyophilized to obtain the pure peptide. ESI-MS m/z [M+1]⁺: 3348.89.

### Example 4: Synthesis of Ac-YGRKKRRQRRRKKNRNKLRRQHSY (SEQ ID NO:7)

The preparation was performed according to the procedure of Example 2, with the exception that Fmoc-N-Me-Tyr(tBu)-OH in step 2.24 was replaced with Fmoc-Tyr(tBu)-OH. After coupling of Fmoc-Tyr(tBu)-OH, deprotection of the Fmoc group, and elution, 30 eq of acetic anhydride,15 eq of pyridine, and 20 mL of *N,N*-dimethylformamide were added, and the mixture was reacted for one hour. The resin was washed with *N,N*-dimethylformamide six times, shrunk with methanol three times (20 mL, 3 minutes each time), and dried under vacuum to obtain the peptide resin.

### 4.1 Cleavage of the Peptide Resin

An ice-cold cleavage cocktail (TFA/MPA/TIS/H₂O = 90:5:2.5:2.5), in a volume equivalent to 10 times the weight of the peptide resin, was added to the flask containing the peptide resin. The reaction was carried out at a constant temperature of 25 °C for 2 hours. After filtration, the filtrate was added to ice-cold methyl tert-butyl ether with a volume of 10 equivalents relative to the cleavage cocktail, and precipitated for 10 minutes, followed by centrifugation. The precipitate was washed repeatedly with room-temperature methyl tert-butyl ether four times and dried under vacuum (< 50 °C) for 3 hours to obtain the crude peptide.

### 4.2 Purification

Mobile phase: water containing 0.1% trifluoroacetic acid/acetonitrile containing 0.1% trifluoroacetic acid; preparative column: ODS-PK C18, 30 mm × 250 mm × 8 mm; flow rate: 25 mL/min; column temperature: 25 °C; wavelength: 220 nm.

Gradient: water/acetonitrile: 5:95 to 95:5, 40 min.

The collected sample was concentrated under reduced pressure and lyophilized to obtain the pure peptide. ESI-MS m/z [M+1]⁺: 3310.93.

### Example 5: Synthesis of C₁₅H₃₁CO-YGRKKRRQRRRKKNRNKLRRQHSY (SEQ ID NO:8)

The preparation was performed according to the procedure of Example 2, except that Fmoc-N-Me-Tyr(tBu)-OH in step 2.24 was replaced with Fmoc-Tyr(tBu)-OH. After coupling of Fmoc-Tyr(tBu)-OH, deprotection of the Fmoc group, and elution, palmitic acid (4 eq), HOBt (4 eq) and DIC (6 eq) were added to the peptide reaction column, followed by 20 mL of *N,N-*dimethylformamide. The reaction was allowed to proceed at a constant temperature of 30 °C for 2 hours. The solution was removed by suction, and the resin was washed with *N,N*-dimethylformamide three times (20 mL each time). A small amount of resin was taken for ninhydrin test. The resin was the transparent and colorless, indicating complete reaction. The resin was shrunk with methanol three times (20 mL, 3 minutes each time) and dried under vacuum to obtain the peptide resin.

### 5.1 Cleavage of the Peptide Resin

An ice-cold cleavage cocktail (TFA/MPA/TIS/H₂O = 90:5:2.5:2.5), in a volume equivalent to 10 times the weight of the peptide resin, was added to the flask containing the peptide resin. The reaction was carried out at a constant temperature of 25 °C for 2 hours. After filtration, the filtrate was added to ice-cold methyl tert-butyl ether with a volume of 10 equivalents relative to the cleavage cocktail, and precipitated for 10 minutes, followed by centrifugation. The precipitate was washed repeatedly with room-temperature methyl tert-butyl ether four times and dried under vacuum (< 50 °C) for 3 hours to obtain the crude peptide.

### 5.2 Purification

Mobile phase: water containing 0.1% trifluoroacetic acid/acetonitrile containing 0.1% trifluoroacetic acid; preparative column: ODS-PK C18, 30 mm × 250 mm × 8 mm; flow rate: 25 mL/min; column temperature: 25°C; wavelength: 220 nm.

Gradient: water/acetonitrile: 5:95 to 95:5, 40 min.

The collected sample was concentrated under reduced pressure and lyophilized to obtain the pure peptide. ESI-MS m/z [M+1]⁺: 3507.15.

### Example 6: Synthesis of YGRKKRRQRRRKKNRNKLRRQHSY-NH₂(SEQ ID NO:9)

Taking a synthesis scale of 1 mmol as an example.

### 6.1 Coupling of Fmoc-Tyr(tBu)-OH

The peptide reaction column was maintained at a constant temperature of 30°C. 3.0 g of Rink-Amide-AM-Resin with a substitution degree of 0.338 mmol/g were weighed and added to the peptide reaction column. The resin was washed once with 10 mL of *N,N*-dimethylformamide, then swelled with 20 mL of dichloromethane for 20 minutes. The solution was removed by suction and deprotection was performed twice with 20% piperidine/*N,N*-dimethylformamide solution (20 mL each time) for 5 and 10 minutes, respectively. After completion, the resin was washed six times with *N,N*-dimethylformamide (20 mL per wash) and dried under vacuum to obtain the peptide resin. A small amount of the resin was taken for ninhydrin test. The resin turned blue-black, indicating complete deprotection.

Fmoc-Tyr(tBu)-OH (4 eq), HOBt (4 eq), and DIC (6 eq) were weighed and added to a peptide reaction column, followed by 20 mL of *N,N*-dimethylformamide. The reaction was allowed to proceed for 2 hours at a constant temperature of 30°C. The solution was removed by suction and then the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide, 20 mL each time. A small amount of resin was taken for ninhydrin test. The resin turned blue-black, indicating complete deprotection.

Steps 6.2 to 6.24 were performed according to the procedure of steps 2.2 to 2.24 of Example 2, except that TBTU in step 2.2 was replaced with HOBt, DIPEA in step 2.2 was replaced with DIC, and Fmoc-N-Me-Tyr(tBu)-OH in Step 2.24 was replaced with Fmoc-Tyr(tBu)-OH.

### 6.25 Cleavage of Peptide Resin

An ice-cold cleavage cocktail (TFA/MPA/TIS/H₂O = 90:5:2.5:2.5), in a volume equivalent to 10 times the weight of the peptide resin, was added to the flask containing the peptide resin. The reaction was carried out at a constant temperature of 25 °C for 2 hours. After filtration, the filtrate was added to ice-cold methyl tert-butyl ether with a volume of 10 equivalents relative to the cleavage cocktail, and precipitated for 10 minutes, followed by centrifugation. The precipitate was washed repeatedly with room-temperature methyl tert-butyl ether four times and dried under vacuum (< 50 °C) for 3 hours to obtain the crude peptide.

### 6.26 Purification

Mobile phase: water containing 0.1% trifluoroacetic acid/acetonitrile containing 0.1% trifluoroacetic acid; preparative column: ODS-PK C18, 30 mm × 250 mm × 8 mm; flow rate: 25 mL/min; column temperature: 25°C; wavelength: 220 nm.

### Gradient: water/acetonitrile: 5:95 to 95:5, 40 min.

The collected sample was concentrated under reduced pressure and lyophilized to obtain the pure peptide. ESI-MS m/z [M+1]⁺: 3267.94.

### Example 7: Synthesis of Ac-YGRKKRRQRRRKKNRNKLRRQHSY-NH₂(SEQ ID NO:10)

### 7.1 Acetylation

Using the product from step 6.24 of Example 6 as the starting material, 30 eq of acetic anhydride, 15 eq of pyridine, and 20 mL of *N,N*-dimethylformamide were added, and the mixture was reacted for one hour. The resin was washed six times with *N,N*-dimethylformamide, shrunk with methanol three times (20 mL, 3 minutes each time), and dried under vacuum to obtain the peptide resin.

### 7.2 Cleavage of the Peptide Resin

An ice-cold cleavage cocktail (TFA/MPA/TIS/H₂O = 90:5:2.5:2.5), in a volume equivalent to 10 times the weight of the peptide resin, was added to the flask containing the peptide resin. The reaction was carried out at a constant temperature of 25 °C for 2 hours. After filtration, the filtrate was added to ice-cold methyl tert-butyl ether with a volume of 10 equivalents relative to the cleavage cocktail, and precipitated for 10 minutes, followed by centrifugation. The precipitate was washed repeatedly with room-temperature methyl tert-butyl ether four times and dried under vacuum (< 50 °C) for 3 hours to obtain the crude peptide.

### 7.3 Purification

Mobile phase: water containing 0.1% trifluoroacetic acid/acetonitrile containing 0.1% trifluoroacetic acid; preparative column: ODS-PK C18, 30 mm × 250 mm × 8 mm; flow rate: 25 mL/min; column temperature: 25°C; wavelength: 220 nm.

Gradient: Water/acetonitrile: 5:95 to 95:5, 40 minutes.

The collected sample was concentrated under reduced pressure and lyophilized to obtain the pure peptide. ESI-MS m/z [M+1]⁺: 3309.95.

### Example 8: Synthesis of pYGRKKRRQRRRKKNRNKLRRQHSY-NH₂(SEQ ID NO:11)

### 8.1 Coupling of Fmoc-Tyr(H₂PO₃BZL)-OH

Using the product from step 6.23 of Example 6 as the starting material, Fmoc-Tyr(H₂PO₃BZL)-OH (4 eq), HOBt (4 eq), and DIC (6 eq) were weighed and added to a peptide reaction column, followed by 20 mL of *N,N*-dimethylformamide. The reaction was allowed to proceed for 2 hours at a constant temperature of 30°C. The solution was removed by suction and then the resin was washed three times with *N,N*-dimethylformamide (20 mL per wash). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide (20 mL each time). A small amount of resin was taken for ninhydrin test. The resin turned blue-black, indicating complete deprotection. The resin was shrunk with methanol three times (20 mL, 3 minutes each time), and dried under vacuum to obtain the crude peptide resin.

### 8.2 Cleavage of the Peptide Resin

An ice-cold cleavage cocktail (TFA/MPA/TIS/H₂O = 90:5:2.5:2.5), in a volume equivalent to 10 times the weight of the peptide resin, was added to the flask containing the peptide resin. The reaction was carried out at a constant temperature of 25 °C for 2 hours. After filtration, the filtrate was added to ice-cold methyl tert-butyl ether with a volume of 10 equivalents relative to the cleavage cocktail, and precipitated for 10 minutes, followed by centrifugation. The precipitate was washed repeatedly with room-temperature methyl tert-butyl ether four times and dried under vacuum (< 50 °C) for 3 hours to obtain the crude peptide.

### 8.3 Purification

Mobile phase: water containing 0.1% trifluoroacetic acid/acetonitrile containing 0.1% trifluoroacetic acid; preparative column: ODS-PK C18, 30 mm × 250 mm × 8 mm; flow rate: 25 mL/min; column temperature: 25 °C; wavelength: 220 nm.

### Gradient: water/acetonitrile: 5:95 to 95:5, 40 min.

The collected sample was concentrated under reduced pressure and lyophilized to obtain the pure peptide. ESI-MS m/z [M+1]⁺: 3347.90.

### Example 9: Synthesis of Me-YGRKKRRQRRRKKNRNKLRRQHSY-NH₂(SEQ ID NO:12)

### 9.1 Coupling of Fmoc-N-Me-Tyr(tBu)-OH

Using the product from step 6.23 of Example 6 as the starting material, Fmoc-N-Me-Tyr(tBu)-OH (4 eq), HOBt (4 eq), and DIC (6 eq) were weighed and added to a peptide reaction column, followed by 20 mL of *N,N*-dimethylformamide. The raction was allowed to proceed for 2 hours at a constant temperature of 30°C. The solution was removed by suction and then the resin was washed three times with *N,N*-dimethylformamide (20 mL each time). A small amount of resin was taken for ninhydrin test. The resin was transparent and colorless, indicating complete reaction.

The peptide reaction column was maintained at 30°C and deprotection was performed twice with a 20% piperidine/*N,N*-dimethylformamide solution (20 mL each time), for 5 and 10 minutes, respectively. After completion, the resin was washed six times with *N,N*-dimethylformamide, 20 mL each time. A small amount of resin was taken for ninhydrin test. The resin turned blue-black, indicating complete deprotection. The resin was shrunk three times with methanol (20 mL each time for 3 minutes) and dried under vacuum to obtain the peptide resin.

### 9.2 Cleavage of the Peptide Resin

An ice-cold cleavage cocktail (TFA/MPA/TIS/H₂O = 90:5:2.5:2.5), in a volume equivalent to 10 times the weight of the peptide resin, was added to the flask containing the peptide resin. The reaction was carried out at a constant temperature of 25 °C for 2 hours. After filtration, the filtrate was added to ice-cold methyl tert-butyl ether with a volume of 10 equivalents relative to the cleavage cocktail, and precipitated for 10 minutes, followed by centrifugation. The precipitate was washed repeatedly with room-temperature methyl tert-butyl ether four times and dried under vacuum (< 50 °C) for 3 hours to obtain the crude peptide.

### 9.3 Purification

Mobile phase: water containing 0.1% trifluoroacetic acid/acetonitrile containing 0.1% trifluoroacetic acid; preparative column: ODS-PK C18, 30 mm × 250 mm × 8 mm; flow rate: 25 mL/min; column temperature: 25°C; wavelength: 220 nm.

### Gradient: water/acetonitrile: 5:95 to 95:5, 40 min.

The collected sample was concentrated under reduced pressure and lyophilized to obtain the pure peptide. ESI-MS m/z [M+1]⁺: 3281.95.

### Example 10: Synthesis of C₁₅H₃₁CO-YGRKKRRQRRRKKNRNKLRRQHSY-NH₂(SEQ ID NO:13)

### 10.1 Palmitoylation

Using the product from step 6.24 of Example 6 as the starting material, palmitic acid (4 eq), HOBt (4 eq), and DIC (6 eq) were added to a peptide reaction column followed by 20 mL of *N,N*-dimethylformamide. The reaction was allowed to proceed for 2 hours at a constant temperature of 30°C. The solution was removed by suction, and the resin was washed three times with *N,N*-dimethylformamide (20 mL each time). A small amount of resin was taken for ninhydrin test. The resin was transparent and colorless, indicating complete reaction. The resin was washed six times with *N,N*-dimethylformamide (20 mL each time), then shrunk three times with methanol (20 mL each time for 3 minutes), and finally dried under vacuum to obtain the peptide resin.

### 10.2 Cleavage of the Peptide Resin

An ice-cold cleavage cocktail (TFA/MPA/TIS/H₂O = 90:5:2.5:2.5), in a volume equivalent to 10 times the weight of the peptide resin, was added to the flask containing the peptide resin. The reaction was carried out at a constant temperature of 25 °C for 2 hours. After filtration, the filtrate was added to ice-cold methyl tert-butyl ether with a volume of 10 equivalents relative to the cleavage cocktail, and precipitated for 10 minutes, followed by centrifugation. The precipitate was washed repeatedly with room-temperature methyl tert-butyl ether four times and dried under vacuum (< 50 °C) for 3 hours to obtain the crude peptide.

### 10.3 Purification

Mobile phase: water containing 0.1% trifluoroacetic acid/acetonitrile containing 0.1% trifluoroacetic acid; preparative column: ODS-PK C18, 30 mm × 250 mm × 8 mm; flow rate: 25 mL/min; column temperature: 25°C; wavelength: 220 nm.

### Gradient: water/acetonitrile: 5:95 to 95:5, 40 min.

The collected sample was concentrated under reduced pressure and lyophilized to obtain the pure peptide, ESI-MS m/z [M+1]⁺: 3506.17.

### Example 11: Synthesis of YGRKKRRQRRRKKNRNKIRRQHSY (SEQ ID NO: 14)

The preparation was performed according to the procedure of Example 1, except that Fmoc-Leu-OH in preparation step 7 was replaced with Fmoc-Ile-OH. A pure peptide was obtained, with ESI-MS m/z [M+1]⁺: 3268.92.

### Example 12: Synthesis of YGRKKRRQRRRKKNRNKIRRQHSY-NH₂ (SEQ ID NO:15)

The preparation was performed according to the procedure of Example 6, except that Fmoc-Leu-OH in preparation steps 6.7 was replaced with Fmoc-Ile-OH. A pure peptide was obtained, with an ESI-MS m/z [M+1]⁺: 3267.94.

### Example 13: Synthesis of YGRKKRRQRRRKKDRDKLRRQHSY (SEQ ID NO:16)

The preparation was performed according to the procedure of Example 1, except that Fmoc-Asn(Trt)-OH in steps 9 and 11 of Example 1 was replaced with Fmoc-Asp(otBu)-OH. A pure peptide was obtained, with ESI-MS m/z [M+1]⁺: 3270.89.

### Example 14: Synthesis of YGRKKRRQRRRK(N-AEEA-Palmitic acid) KNRNKLRRQHSY (SEQ ID NO:17)

Taking a synthesis scale of 1 mmol as an example. The synthesis procedure follows that of Example 1, with the exception that Fmoc-Lys(Boc)-OH in step 13 of Example 1 is replaced with Fmoc-Lys(Dde)-OH.

### 14.1 Coupling of Fmoc-Lys(Dde)-OH

Fmoc-Lys(Dde)-OH (4 eq), HOBt (4 eq), and DIC (6 eq) were weighed and added to a peptide reaction column, followed by 20 mL of *N,N*-dimethylformamide. The reaction was allowed to proceed for 2 hours at a constant temperature of 30°C. The solution was removed by suction and then the resin was washed three times with *N,N*-dimethylformamide (20 mL each time). A small amount of resin was taken for ninhydrin test. The resin was transparent and colorless, indicating complete reaction.

The Dde protecting group was removed with 20 mL of a 5% hydrazine hydrate/DMF solution for 60 minutes, with the peptide reaction column maintained at a constant temperature of 30 °C throughout. After completion, the resin was washed six times with DMF, 20 mL each time. The peptide reaction column at a constant temperature of 30°C during the reaction and washing processes. A small amount of resin was taken for ninhydrin test. The resin turned blue-black, indicating complete deprotection.

### 14.2 Coupling of Fmoc-AEEA-OH

4 eq of Fmoc-AEEA-OH, 4 eq of HOBT, and 6 eq of DIC were weighed and added to a peptide reaction column, followed by 20 mL of DMF. The reaction was allowed to proceed for two hours at a constant temperature of 30°C. Then the solution was removed by suction and then the resin was washed three times with DMF, 20 mL each time. A small amount of resin was taken for ninhydrin test. The resin was transparent and colorless, indicating complete reaction.

The peptide reaction column was maintained at a constant temperature of 30°C. Fmoc protecting group was removed twice using a 20% piperidine/DMF solution (20 mL each time), for 5 and 10 minutes, respectively. After completion, the resin was washed six times with DMF (20 mL each time) and dried under vacuum to obtain the peptide resin. A small amount of the resin was taken for ninhydrin test. The resin turned blue-black, indicating complete deprotection.

### 14.3 Palmitoylation

Palmitic acid (4 eq), HOBt (4 eq), and DIC (6 eq) were added to the peptide reaction column, followed by 20 mL of *N,N*-dimethylformamide. The reaction was allowed to proceed for two hours at a constant temperature of 30°C.The solution was removed by suction and then the resin was washed the resin three times with *N,N*-dimethylformamide (20 mL each time; ). A small amount of resin was taken for ninhydrin test. The resin was transparent and colorless, indicating complete reaction. The resin was washed six times with *N,N*-dimethylformamide (20 mL each time), then shrunk three times with methanol (20 mL each time for 3 minutes), and dried under vacuum to obtain the peptide resin.

### 14.4 Cleavage of the Peptide Resin

An ice-cold cleavage cocktail (TFA/MPA/TIS/H₂O = 90:5:2.5:2.5), in a volume equivalent to 10 times the weight of the peptide resin, was added to the flask containing the peptide resin. The reaction was carried out at a constant temperature of 25 °C for 2 hours. After filtration, the filtrate was added to ice-cold methyl tert-butyl ether with a volume of 10 equivalents relative to the cleavage cocktail, and precipitated for 10 minutes, followed by centrifugation. The precipitate was washed repeatedly with room-temperature methyl tert-butyl ether four times and dried under vacuum (< 50 °C) for 3 hours to obtain the crude peptide.

### 14.5 Purification

Mobile phase: water containing 0.1% trifluoroacetic acid/acetonitrile containing 0.1% trifluoroacetic acid; preparative column: ODS-PK C18, 30 mm × 250 mm × 8 mm; flow rate: 25 mL/min; column temperature: 25°C; wavelength: 220 nm.

Gradient: water/acetonitrile: 5:95 to 95:5, 40 min.

The collected sample was concentrated under reduced pressure and lyophilized to obtain the pure peptide. ESI-MS m/z [M+1]⁺: 3652.2251.

### Example 15: Synthesis of YGRKKRRQRRRKKNRNKLRREHSY (SEQ ID NO:18)

The preparation was performed according to the procedure of Example 1, except that Fmoc-Gln(Trt)-OH in step 4 of Example 1 was replaced with Fmoc-Glu(otBu)-OH. A pure peptide was obtained, with ESI-MS m/z [M+1]⁺: 3269.91.

### Example 16: Synthesis of YGRKKRRQRRRKKNRDKLRREHSY (SEQ ID NO:19)

The preparation was performed according to the procedure of Example 1, except that Fmoc-Gln(Trt)-OH in step 4 and Fmoc-Asn(Trt)-OH in step 9 of Example 1 were replaced with Fmoc-Glu(otBu)-OH and Fmoc-Asp(otBu)-OH, respectively.A pure peptide was obtained, with ESI-MS m/z [M+1]⁺: 3270.89.

### Example 17: Synthesis of YGRKKRRERRRKKDRNKLRRQHSY (SEQ ID NO:20)

The preparation was performed according to the procedure of Example 1, except that Fmoc-Asn(Trt)-OH in step 11 and Fmoc-Gln(Trt)-OH in step 17 of Example 1 were replaced with Fmoc-Asp(otBu)-OH and Fmoc-Glu(otBu)-OH, respectively.A pure peptide was prepared, with ESI-MS m/z [M+1]⁺: 3270.89.

### Example 18: Synthesis of Ac-YGRKKRRERRRKKDRDKLRREHSY-NH₂(SEQ ID NO:21)

The preparation was performed according to the procedure of Example 7, except that Fmoc-Gln(Trt)-OH in step 7.4, Fmoc-Asn(Trt)-OH in step 7.9, Fmoc-Asn(Trt)-OH in step 11, and Fmoc-Gln(Trt)-OH in step 17 were replaced with Fmoc-Glu(otBu)-OH,Fmoc-Asp(otBu)-OH, Fmoc-Asp(otBu)-OH, and Fmoc-Glu(otBu)-OH, respectively. A pure peptide was obtained, with ESI-MS m/z [M+1]⁺: 3314.89.

### Example 19: Synthesis of Me-YGRKKRRERRRKKDRDKLRREHSY-NH₂(SEQ ID NO:22)

The preparation was performed according to the procedure of Example 9, except that Fmoc-Gln(Trt)-OH in step 9.4, Fmoc-Asn(Trt)-OH in step 9.9, Fmoc-Asn(Trt)-OH in step 9.11, and Fmoc-Gln(Trt)-OH in step 9.17 were replaced with Fmoc-Glu(otBu)-OH, Fmoc-Asp(otBu)-OH, Fmoc-Asp(otBu)-OH, and Fmoc-Glu(otBu)-OH, respectively.A pure peptide was obtained, with ESI-MS m/z [M+1]⁺: 3286.89.

### Example 20: Synthesis of Ac-YGRKKRRERRRKKDRDKlRREHSY-NH₂(SEQ ID NO:23)

The preparation was performed according to the procedure of Example 18, except that Fmoc-Leu-OH in step 18.7 was replaced with Fmoc-Leu-OH (D-configuration). A pure peptide was prepared, with ESI-MS m/z [M+1]⁺: 3314.89.

### Example 21: Synthesis of Ac-YGRKKRRERRRKKDRDKLRRQHSY-NH₂(SEQ ID NO:24)

The preparation was performed according to the procedure of Example 7, except that Fmoc-Asn(Trt)-OH in step 7.9, Fmoc-Asn(Trt)-OH in step 7.11, and Fmoc-Gln(Trt)-OH in step 7.17 were replaced with Fmoc-Asp(otBu)-OH, Fmoc-Asp(otBu)-OH, and Fmoc-Glu(otBu)-OH, respectively. A pure peptide was obtained, with ESI-MS m/z [M+1]⁺: 3313.90.

### Example 22: Synthesis of Me-YGRKKRRERRRKKDRDKLRRQHSY-NH₂(SEQ ID NO:25)

The preparation was performed according to the procedure of Example 9, except that Fmoc-Asn(Trt)-OH in step 9.9, Fmoc-Asn(Trt)-OH in step 9.11, and Fmoc-Gln(Trt)-OH in step 9.17 were replaced with Fmoc-Asp(otBu)-OH, Fmoc-Asp(otBu)-OH, and Fmoc-Glu(otBu)-OH, respectively. A pure peptide was obtained, with ESI-MS m/z [M+1]⁺: 3285.91.

### Example 23: Synthesis of Ac-YGRKKRRERRRKKDRDKVRREHSY-NH₂(SEQ ID NO:26)

The preparation was performed according to the procedure of Example 18, except that Fmoc-Leu-OH in step 18.7 is replaced with Fmoc-Val-OH. A pure peptide was obtained, with ESI-MS m/z [M+1]⁺: 3300.87.

### Example 24: Synthesis of Me-YAibRKKRRERRRKKDRDKTleRREHSY-NH₂(SEQ ID NO:27)

The preparation was performed according to the procedure of Example 19, except that Fmoc-Leu-OH in step 19.7 and Fmoc-Gly-OH in step 19.23 were replaced with Fmoc-Tle-OH and Fmoc-Aib-OH, respectively. A pure peptide was prepared, with ESI-MS m/z [M+1]⁺: 3313.92.

### Example 25: Synthesis of Ac-YGRKKRRERRRKKDRDKTleRREHSY-NH₂(SEQ ID NO:28)

The preparation was performed according to the procedure of Example 18, except that Fmoc-Leu-OH in step 18.7 was replaced with Fmoc-Tle-OH. A pure peptide was prepared, with an ESI-MS m/z [M+1]⁺: 3313.88.

### Example 26: Synthesis of Ac-YGRK(D-K)(D-R)RER(D-R)RKKDRDK(D-L)(D-R)REHSY-NH₂(SEQ ID NO:29)

The preparation was performed according to the procedure of Example 18, except that Fmoc-Arg(Pbf)-OH in step 18.6, Fmoc-Leu-OH in step 18.7, Fmoc-Arg(Pbf)-OH in step 18.15, Fmoc-Arg(Pbf)-OH in step 18.19, and Fmoc-Lys(Boc)-OH in step 18.20 were replaced with Fmoc-Arg(Pbf)-OH (D-configuration), Fmoc-Leu-OH (D-configuration), Fmoc-Arg(Pbf)-OH (D-configuration), Fmoc-Arg(Pbf)-OH (D-configuration), and Fmoc-Lys(Boc)-OH (D-configuration), repectively. A pure peptide was prepared, with ESI-MS m/z [M+1]⁺: 3312.89.

### Experimental Example 1: The polypeptide compound according to the present application increases the cell viability of PC-12 neural cells under glutamate stimulation

### 1.1. Culture of PC-12 cell line and establishment of excitotoxic cell death model

PC-12 cells (a neuroblastoma cell line derived from rat adrenal medulla) were cultured in DMEM/F12 complete medium until eaching 80-90% confluence. The cells were then digested, collected, resuspended, and seeded at density of 5 × 10⁴ cells per well in a 24-well plate precoated with coverslips. Following 26 hours of cell culture, excitotoxic cell death was induced by glutamate stimulation. For model establishment: an appropriate amount of glutamate was weighed and dissolved in DMEM/F12 medium to prepare a stock solution. After filtration, the stock solution was diluted with culture medium and administered to the cells via medium replacement. The cells were further incubated for 24 hours after administration.

### 1.2 Administration of small molecules

Prior to glutamate modeling, cells incubated as described in the previous step were pretreated with 10 nM of the polypeptide compound according to the present application for 30 minutes, followed by glutamate stimulation.

### 1.3. Propidium iodide staining

Cells treated with the polypeptide compound according to the present application were stained with propidium iodide to assess cell death. After discarding the culture medium, the cells were washed with PBS, and propidium iodide/Hoechst was added. After incubating for 30 minutes, the cells were removed and the staining solution was discarded. After washing with PBS, the cells were fixed and mounted, and subjected to image acquisition under a fluorescence microscope. The number of propidium iodide-positive cells and Hoechst-positive cells in the acquired images was counted to calculate the cell viability. MK-801 was used as the positive control compound. The normalized cell viability was calculated using the following formula: (cell viability of the compound group - cell viability of the glutamate group) / (cell viability of the positive control group - cell viability of the glutamate group) × 100%. Detailed data are presented in Table 3.

Based on the above dose-response studies of the compound, the inhibitory effects of different small-molecule compounds at the same dose (10 nM) against excitotoxic death in PC-12 neurons cells were further compared. Detailed data are shown in Table 3.

**Table 3: Normalized cell viability (%) of PC-12 neural cell promoted by various polypeptide compounds**

| **Example** | **Normalized cell viability (%)** |
|---|---|
| Example 1 | 41.00% |
| Example 2 | 65.56% |
| Example 4 | 85.00% |
| Example 9 | 63.17% |
| Example 10 | 49.46% |
| Example 12 | 58.59% |
| Example 14 | 84.14% |
| Example 16 | 85.15% |
| Example 18 | 87.20% |
| Example 19 | 87.71% |
| Example 20 | 87.36% |
| Example 21 | 50.61% |
| Example 22 | 79.97% |
| Example 23 | 74.16% |
| Example 24 | 85.77% |
| Example 25 | 86.80% |
| Example 26 | 82.39% |

The results indicated that, compared to Example 1, the polypeptide compounds according to the present application effectively inhibit glutamate-induced excitotoxic death of PC-12 cells, demonstrating a significant advantage. This indicates that the biological activity of the polypeptide compounds according to the present application is significantly improved. Based on this, the dose-response relationship of the neuroprotective effect of the polypeptide compounds according to the present application on PC-12 neural cells was analyzed. Detailed data are shown in Table 4.

**Table 4: Study of the dose-response relationship of various polypeptide compounds in promoting the viability of PC-12 neurons**

| **Example** | **EC_{50 (}nM)** |
|---|---|
| Example 1 | 23.59 |
| Example 2 | 12.61 |
| Example 6 | 13.5 |
| Example 7 | 8.72 |
| Example 9 | 4.83 |
| Example 16 | 18.77 |
| Example 24 | 1.354 |

The results indicate that the polypeptide compounds according to the present application can effectively inhibit glutamate-induced excitotoxic death of PC-12 cells. Compared with Example 1, their EC₅₀ values were significantly decreased, indicating that the biological activity of the polypeptide compounds according to the present application is significantly improved.

### Experimental Example 2: Pharmacokinetic characteristics of various compounds in Rats

Intravenous administration: Three SD rats per group were administered the compounds of Example 1, Example 2, Example 3, Example 6, Example 7, Example 17, and Example 19 via the tail vein, respectively, at a dose of 10 mg/kg. all SD rats were allowed free access to food and water after administration. Plasma was collected 3 min, 10 min, 30 min, 1 h, 2 h, 6 h, 10 h, and 24 h after administration, with 3 rats per time point.

Sample processing: Whole blood was anticoagulated with EDTA-K₂. Within 30 minutes of collection, the blood was centrifuged at 3000g for 10 minutes at 2-8°C. After centrifugation, the separated plasma was transferred to new 1.5 mL centrifuge tubes and stored at -80°C.

Analytical method: The concentrations of each compounds in rat plasma were determined by LC-MS/MS using Shimadzu LC30AD and AB Sciex QTRAP 6500+instruments. The relevant pharmacokinetic parameters after administration were calculated. The experimental results are shown in Table 5.

**Table 5: Pharmacokinetic parameters after administration**

| Example | Vₛₛ (L/kg) | T_{1/2 (}h) | MRT_{inf}(h) |
|---|---|---|---|
| Example 1 | 1.29 | 0.76 | 0.87 |
| Example 2 | 3.94 | 1.73 | 1.84 |
| Example 3 | 7.90 | 1.28 | 1.71 |
| Example 6 | 2.53 | 1.23 | 1.65 |
| Example 7 | 3.58 | 1.51 | 1.93 |
| Example 17 | 23.1 | 1.66 | 2.29 |
| Example 19 | 9.11 | 1.16 | 1.73 |

The results indicate that the polypeptide compound according to the present application can effectively improve the stability and cellular permeability of the peptide itself in vivo. Compared with Example 1, the plasma half-life (T_{1/2}) is significantly prolonged, the volume of distribution at steady state (Vₛₛ) was significantly increased, and the mean residence time (MRT_{inf}) in vivo was is significantly prolonged, indicating that the polypeptide compounds according to the present application can better distribute from blood to tissues and exert their effects in vivo for a longer duration.

Although various embodiments of the compositions and methods for treating nerve injury have been described in detail herein, such embodiments are provided merely as non-limiting examples of the subject matter described herein. Accordingly, those skilled in the art will appreciate that various modifications and changes may be made to the present application without departing from the scope thereof. Indeed, the subject matter of the present application is not intended to be exhaustive or to limit the scope of the application.

Furthermore, in the description of the representative embodiments, the present application has described the methods and/or processes of the present application in a specific sequence of steps. However, the method or process should not be limited to the specific sequence of steps described. Other sequences of steps are possible. Therefore, the specific sequence of steps described herein should not be construed as a limitation on the present application. Furthermore, the disclosure regarding the method and/or process should not be limited to performing their steps in the described order. Such an order may vary and still fall within the scope of the present application.

The above are merely specific embodiments of the present application, enabling those skilled in the art to understand or implement the present application. Various modifications to these embodiments will be readily apparent to those skilled in the art, and the general principles defined herein may be implemented in other embodiments without departing from the spirit or scope of the present application. Therefore, the present application shall not be limited to the embodiments described herein but shall be accorded the broadest scope consistent with the principles and novel features disclosed herein.

## Claims

1. A polypeptide compound comprising a structure represented by General Formula (I):
KX²X³RX⁵KX⁷X⁸RX¹⁰HSY (SEQ ID NO:3)-R¹ (I),
wherein X² is selected from the group consisting of Lys, HoLys, Dap, DAB, Cit, Orn, D-Lys, D-HoLys, D-Dap, D-DAB, D-Cit, D-Orn, and Lys whose side-chain amino group is modified by Z¹; X³and X⁵are each independently selected from the group consisting of Asn, Asp, D-Asn, and D-Asp; X⁷ is selected from the group consisting of Ile, Leu, Val, Tel, D-Ile, D-Leu, D-Val, and D-Tel; X⁸ is selected from the group consisting of Arg, HomoArg, D-Arg, and D-HomoArg; X¹⁰ is selected from the group consisting of Glu, Gln, D-Glu, and D-Gln;
R¹ is selected from the group consisting of -OH, -ol, -NR²R³, and -OR⁴, wherein R² and R³ are each independently selected from the group consisting of hydrogen, C₁₋₁₆ alkyl, C₃₋₁₂ cycloalkyl, and 3- to 12-membered heterocycloalkyl, or R² and R³ together with a nitrogen atom to which R² and R³ are attached form a 3- to 12-membered heterocycloalkyl; R⁴ is selected from the group consisting of C₁₋₁₆ alkyl, C₃₋₁₂ cycloalkyl, and 3- to 12-membered heterocycloalkyl;
the alkyl, cycloalkyl, and heterocycloalkyl are each optionally substituted by one or more identical or different R⁵;
R⁵ is selected from the group consisting of halogen, nitro, cyano, hydroxy, carbonyl, carboxyl, -NRₐR_{b}, thiol, C₁₋₈ alkyl, C₁₋₈ alkoxy, halo C₁₋₈ alkyl, halo C₁₋₈ alkoxy, C₃₋₈ cycloalkyl, C₃₋₈ cycloalkoxy, 3- to 8-membered heterocycloalkyl, -SC₁₋₃ alkyl, -SO₂C₁₋₃ alkyl, -SO₂NRₐR_{b}, - S(O)C₁₋₃ alkyl, -C(O)NRₐR_{b}, -C(O)C₁₋₃ alkyl, -C(O)OC₁₋₃ alkyl, -OC(O)C₁₋₃ alkyl, C₆₋₁₀ aryl, and 5- to 10-membered heteroaryl;
Rₐ and R_{b} are each independently hydrogen or C₁₋₈ alkyl; or Rₐ and R_{b} together with a nitrogen atom to which Rₐ and R_{b} are attached form a 3- to 6-membered heterocycloalkyl; and
Z¹is -AEEA-COR⁶, wherein R⁶ is C₁₋₁₅ alkyl.

2. The polypeptide compound according to claim 1, wherein the polypeptide compound further comprises a cell-penetrating peptide at a N-terminus or C-terminus of the polypeptide compound.

3. The polypeptide compound according to claim 2, wherein the cell-penetrating peptide is at the N-terminus of the polypeptide compound, a N-terminus of the cell-penetrating peptide is modified by R⁷, and the structure of the polypeptide compound is represented by General Formula (II):
R⁷-cell-penetrating peptide-KX²X³RX⁵KX⁷X⁸RX¹⁰HSY (SEQ ID NO:3)-R¹ (II),
wherein R⁷ is selected from the group consisting of hydrogen, C₁₋₁₆ alkyl, C₃₋₁₂ cycloalkyl, 3-to 12-membered heterocycloalkyl, R⁸CO-, and phosphate group, wherein R⁸ is selected from the group consisting of C₁₋₁₆ alkyl, C₃₋₁₂ cycloalkyl, 3- to 12-membered heterocycloalkyl, C₆₋₁₀ aryl, and 5- to 10-membered heteroaryl.

4. The polypeptide compound according to claim 2 or 3, wherein the cell-penetrating peptide is selected from the group consisting of a TAT sequence, an MAP sequence, an MTS sequence, an R9 sequence, and a variant thereof.

5. The polypeptide compound according to any one of claims 2 to 4, wherein the cell-penetrating peptide is the TAT sequence or a variant thereof, and the TAT sequence comprises at least an amino acid fragment from positions 49 to 57 of a transcriptional transactivator protein.

6. The polypeptide compound according to any one of claims 2 to 4, wherein the cell-penetrating peptide is the TAT sequence, X² is Lys; X³ and X⁵ are each Asn; X⁷ is Leu; X⁸ is Arg; X¹⁰ is Gln; and the structure of the polypeptide compound is represented by General Formula (III):
R⁷-TAT-KKNRNKLRRQHSY(SEQ ID NO:1)-R¹ (III),
wherein TAT is linked to KKNRNKLRRQHSY (SEQ ID NO:1) via a backbone amide bond, and a nitrogen atom in the backbone amide bond is optionally C₁₋₃-alkylated, such as methylated;
R¹is selected from the group consisting of -OH, -ol, -NR²R³, and -OR⁴, wherein R² and R³ are each independently selected from the group consisting of hydrogen, C₁₋₁₆ alkyl, C₃₋₁₂ cycloalkyl, and 3- to 12-membered heterocycloalkyl, or R² and R³ together with a nitrogen atom to which R² and R³ are attached form a 3- to 12-membered heterocycloalkyl; and R⁴ is selected from the group consisting of C₁₋₁₆ alkyl, C₃₋₁₂ cycloalkyl, and 3- to 12-membered heterocycloalkyl;
the alkyl, cycloalkyl, and heterocycloalkyl are each optionally be substituted by one or more identical or different R⁵;
R⁵ is selected from the group consisting of halogen, nitro, cyano, hydroxy, carbonyl, carboxyl, -NRₐR_{b}, thiol, C₁₋₈ alkyl, C₁₋₈ alkoxy, halo C₁₋₈ alkyl, halo C₁₋₈alkoxy, C₃₋₈ cycloalkyl, C₃₋₈ cycloalkyloxy, 3- to 8-membered heterocycloalkyl, -SC₁₋₃ alkyl, -SO₂C₁₋₃ alkyl, -SO₂NRₐR_{b}, - S(O)C₁₋₃ alkyl, -C(O)NRₐR_{b}, -C(O)C₁₋₃ alkyl, -C(O)OC₁₋₃ alkyl, -OC(O)C₁₋₃ alkyl, C₆₋₁₀ aryl, and 5- to 10-membered heteroaryl;
Rₐ and R_{b} are each independently hydrogen or a C₁₋₈ alkyl; or Rₐ and R_{b} together with a nitrogen atom to which Rₐ and R_{b} are attached form a 3- to 6-membered heterocycloalkyl; and when R⁷ is hydrogen, R¹ is not -OH; the TAT sequence comprises at least the amino acid fragment from positions 49 to 57 of the transcriptional transactivator protein; and
R⁷ is selected from the group consisting of hydrogen, C₁₋₁₆ alkyl, C₃₋₁₂ cycloalkyl, 3- to 12-membered heterocycloalkyl, R⁸CO-, and phosphate group, wherein R⁸ is selected from the group consisting of C₁₋₁₆ alkyl, C₃₋₁₂ cycloalkyl, 3- to 12-membered heterocycloalkyl, C₆₋₁₀ aryl, and 5-to 10-membered heteroaryl.

7. The polypeptide compound according to any one of claims 4 to 6, wherein the TAT sequence is selected from the group consisting of YGRKKRRQRRR (SEQ ID NO:4), GRKKRRQRRR (SEQ ID NO: 33), YGRKKRRQRRRPPQ (SEQ ID NO: 34), and GRKKRRQRRRQ (SEQ ID NO: 35).

8. The polypeptide compound according to claim 6 or 7, wherein the variant of the TAT sequence comprises 1, 2, or 3 amino acid substitutions, additions, or deletions relative to the TAT sequence.

9. A polypeptide compound according to any one of claims 6 to 8, wherein the variant of the TAT sequence comprises a sequence having at least 80%, at least 85%, at least 90%, at least 95%, at least 99%, or 100% identity to YGRKKRRQRRR (SEQ ID NO: 4).

10. The polypeptide compound according to any one of claims 6 to 9, wherein the variant of the TAT sequence is an amino acid sequence formed by replacing at least one Gln in the TAT sequence by Glu, and/or at least one L-Arg in the TAT sequence by D-Arg, and/or at least one L-Lys in the TAT sequence by D-Lys.

11. The polypeptide compound according to any one of claims 1 to 10, wherein R¹ is selected from the group consisting of -OH and -NH₂.

12. The polypeptide compound according to any one of claims 3 to 11, wherein R⁷ is selected from the group consisting of hydrogen, C₁₋₃ alkyl, and acetyl.

13. The polypeptide compound according to any one of claims 1 to 12, wherein the polypeptide compound is selected from the polypeptide compounds as shown in SEQ ID NO:5 to SEQ ID NO:29:
Me-YGRKKRRQRRRKKNRNKLRRQHSY (SEQ ID NO:5),
pYGRKKRRQRRRKKNRNKLRRQHSY (SEQ ID NO:6),
Ac-YGRKKRRQRRRKKNRNKLRRQHSY (SEQ ID NO:7),
C₁₅H₃₁CO-YGRKKRRQRRRKKNRNKLRRQHSY (SEQ ID NO:8),
YGRKKRRQRRRKKNRNKLRRQHSY-NH₂ (SEQ ID NO:9),
Ac-YGRKKRRQRRRKKNRNKLRRQHSY-NH₂ (SEQ ID NO:10),
pYGRKKRRQRRRKKNRNKLRRQHSY-NH_{2 (}SEQ ID NO:11),
Me-YGRKKRRQRRRKKNRNKLRRQHSY-NH₂ (SEQ ID NO:12),
C₁₅H₃₁CO-YGRKKRRQRRRKKNRNKLRRQHSY-NH₂(SEQ ID NO:13),
YGRKKRRQRRRKKNRNKIRRQHSY (SEQ ID NO:14),
YGRKKRRQRRRKKNRNKIRRQHSY-NH₂ (SEQ ID NO:15),
YGRKKRRQRRRKKDRDKLRRQHSY (SEQ ID NO:16),
YGRKKRRQRRR-K(N-AEEA-Palmitic acid)-KNRNKLRRQHSY (SEQ ID NO:17),
YGRKKRRQRRRKKNRNKLRREHSY (SEQ ID NO:18),
YGRKKRRQRRRKKNRDKLRREHSY (SEQ ID NO:19),
YGRKKRRERRRKKDRNKLRRQHSY (SEQ ID NO:20),
Ac-YGRKKRRERRRKKDRDKLRREHSY-NH₂ (SEQ ID NO:21),
Me-YGRKKRRERRRKKDRDKLRREHSY-NH₂ (SEQ ID NO:22),
Ac-YGRKKRRERRRKKDRDK(D-L)RREHSY-NH₂(SEQ ID NO:23),
Ac-YGRKKRRERRRKKDRDKLRRQHSY-NH₂ (SEQ ID NO:24),
Me-YGRKKRRERRRKKDRDKLRRQHSY-NH₂ (SEQ ID NO:25),
Ac-YGRKKRRERRRKKDRDKVRREHSY-NH₂ (SEQ ID NO:26),
Me-YAibRKKRRERRRKKDRDKTleRREHSY-NH₂ (SEQ ID NO:27),
Ac-YGRKKRRERRRKKDRDKTleRREHSY-NH₂ (SEQ ID NO:28) and
Ac-YGRK(D-K)(D-R)RER(D-R)RKKDRDK(D-L)(D-R)REHSY-NH₂ (SEQ ID NO:29).

14. A pharmaceutical composition comprising a polypeptide compound according to any one of claims 1-13, or a variant or a pharmaceutically acceptable salt thereof, and a pharmaceutically acceptable excipient.

15. The use of the polypeptide compound according to any one of claims 1-13, or a variant or pharmaceutically acceptable salt thereof, or the pharmaceutical composition according to claim 14, in the manufacture of a medicament, wherein the medicament is for the treatment of traumatic brain injury and/or for inhibiting neural damage following traumatic brain injury.

16. A method for treating traumatic brain injury and/or inhibiting neural damage following traumatic brain injury, comprising administering to a subject the polypeptide compound according to any one of claims 1-13, or a variant or pharmaceutically acceptable salt thereof, or the pharmaceutical composition according to claim 14.

17. The polypeptide compound according to any one of claims 1 to 13, or a variant or pharmaceutically acceptable salt thereof, or the pharmaceutical composition according to claim 14, for use in the treatment and/or prevention of traumatic brain injury and/or in the inhibition of neural damage following traumatic brain injury.
